# EUROPEAN PATENT APPLICATION

(11) **EP 4 477 651 A1**
(43) Date of publication of application: **18.12.2024**
(21) Application number: 23766094.9
(22) Date of filing: 09.03.2023
(51) Int. Cl.: C07D 307/68, C07D 307/46

(54) **METHOD FOR PREPARING 2,5-FURANDICARBOXYLIC ACID COMPOUND**

(30) Priority: 10.03.2022 CN 202210234062
(71) Applicant: Jiangsu Celluranics New Material Technology Co., Ltd., Taixing Taizhou, Jiangsu 225400 (CN)
(72) Inventor: WANG, Hu, Taizhou, Jiangsu 225400 (CN); GUO, Neng, Taizhou, Jiangsu 225400 (CN)
(74) Representative: Maiwald GmbH
(86) International application number: PCT/CN2023/080541
(87) International publication number: WO 2023/169517

(57) **Abstract**

Discloses in the present invention is a method for preparing a 2,5-furandicarboxylic acid (FDCA) compound. The method comprises: providing a hexose diacid compound, an acid catalyst, and an ionic liquid; and dehydrating the hexose diacid compound in the ionic liquid under the catalysis of the acid catalyst to form the 2,5-furandicarboxylic acid compound; wherein the ionic liquid comprises a cationic moiety selected from pyridinium, pyridazinium, pyrimidinium, pyrazinium, oxazinium, thiazinium, imidazolium, pyrazolium, thiazolium, isothiazolium, oxazolium, isoxazolium, and triazolium, as well as an anionic moiety selected from halogen anions.

## Description

This application claims the priority of Chinese Patent Application No. 202210234062.3 filed on March 10, 2022, the disclosure of which is hereby cited in its entirety as part of the present application.

### INVENTION FIELD

The present disclosure belongs to the field of chemical engineering. In particular, the present disclosure relates to a method for the acid-catalyzed preparation of 2,5-furandicarboxylic acid compound using an ionic liquid as reaction medium.

### BACKGROUND

Polyethylene terephthalates, abbreviated as PET polyesters, are one of the most widely used polymeric materials in modern life. PET polyesters are formed by polycondensation of purified terephthalic acid (PTA) and ethylene glycol which are based on fossil resources. As the environmental pollution, global warming, and resource crisis caused by the use of fossil resources have increasingly become the focus of attention, governments and industries of various major economies in the world have listed the preparation of new materials from renewable biomass resources as a strategic development direction. 2,5-furandicarboxylic acid (FDCA), which is comparable to PTA and can be converted from biomass, is one of the 12 most important platform compounds selected by the U.S. Department of Energy from more than 300 bio-based compounds in 2004. Its molecular structure is similar to that of PTA, both of which have cyclic conjugated systems and contain 2 carboxylic acid groups. Polyethylene furanoates (PEF polyesters) with FDCA and renewable ethylene glycol as raw materials are 100% renewable polyesters, of which the CO₂ emissions corresponding to their full life cycle is 70% less than the CO₂ emission corresponding to the production of PET polyesters. The PEF polyesters have 10 times higher oxygen barrier property and 6-10 times higher carbon dioxide barrier property than traditional PET polyesters, which can greatly extend the shelf life of food and beverage. In addition, the PEF polyesters have better mechanical properties than traditional PET polyesters, and can replace the PET polyesters in many applications such as automobile parts, cables, clothing fabrics, carpets, and films.

In the past two decades, more and more people tried to prepare FDCA from renewable biomass resources (cellulose, starch, glucose, fructose, and the like) to reduce the dependence on traditional fossil energy.

FDCA can be prepared by air oxidation of 5-hydroxylmethyl furfural (HMF), and the catalyst can be Co/Mn/Br homogeneous system (CN102040571A, CN102648191A, CN109651311A) or noble metal heterogeneous system on support (US 10,654,819 B2). However, due to the unstable nature of HMF, the isolation and purification is costly, which leads to the high cost of FDCA prepared by this route.

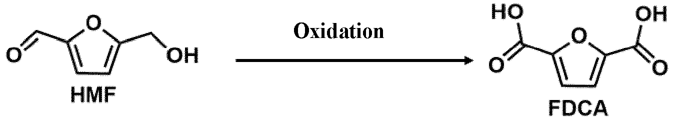

FDCA can also be prepared by dehydration of hexose diacids (WO2017/083297A1, WO2019/014393A1, CN107417651A), which can be glucose diacid, galactose diacid, etc.

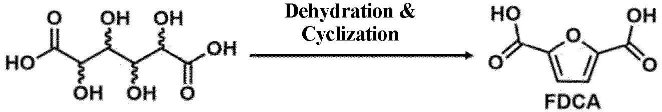

At present, the synthesis of FDCAis mostly focused on the first chemical route, including the dehydration of fructose to produce HMF, followed by the oxidation in the presence of various catalysts to produce FDCA. At present, this route faces two challenges. One is that the intermediate HMF with low volatility and low decomposition temperature is a molecule that is difficult to isolate. The other is that the oxidization of HMF to FDCA is less effective, and needs noble catalyst, low concentration reaction condition, and control of pH. Due to these challenges, there is no commercial production from HMF to FDCA at present.

Moreover, FDCA can also be prepared by dehydration and cyclization of hexose diacids. However, traditional methods involving the acid-catalyzed dehydration by sulfuric acid (H₂SO₄), hydrobromic acid (HBr), methanesulfonic acid (MSA), trifluoromethanesulfonic acid (TfOH), or the like has low yield, which needs to promote the reaction and improve the yield of FDCA by adding excess of halides such as LiBr and NaBr. However, the reaction in the presence of halides such as LiBr and NaBr often results in the production of pressure in the reaction system due to the generation of HX at high temperature, and moreover byproducts such as HBr put forward extremely strict requirements for the material selection of the reaction equipment.

US9,701,652B2 reported the production of FDCA by sulfuric acid-catalyzed dehydration of saccharic acid or galactaric acid in an ionic liquid of 1-butyl-3-methyl-imidazole bisulfate, which had low FDCA yield, although no excess of halide was added. The maximum yield as reported was only 52%, and in most experiments, the FDCA yield was around 0-30%.

Therefore, there is still a need for an improved method for preparing FDCA in the market to overcome one or more of the above disadvantages in the prior art.

### SUMMARY

To overcome one or more disadvantages in the prior art, an obj ect of the present disclosure is to provide an improve method for preparing a 2,5-furandicarboxylic acid (FDCA) compound. The inventive method can provide one or more of the following advantages: being carried out at ambient pressure; mild reaction conditions; good selectivity of FDCA; high yield of FDCA; short reaction time period; high purification of the prepared products; simple operation; and less pollution to the environment during the whole production process.

The present disclosure provides a method for preparing a 2,5-furandicarboxylic acid (FDCA) compound comprising: providing a hexose diacid (aldaric acid) compound, an acid catalyst, and an ionic liquid; and dehydrating the hexose diacid compound in the ionic liquid in the presence of the acid catalyst to form the 2,5-furandicarboxylic acid compound; wherein the ionic liquid comprises a cationic moiety selected from the group consisting of pyridinium, pyridazinium, pyrimidinium, pyrazinium, oxazinium, thiazinium, imidazolium, pyrazolium, thiazolium, isothiazolium, oxazolium, isoxazolium, and triazolium, and an anionic moiety selected from halogen anions.

For example, the ionic liquid has a substituent selected from the group consisting of linear or branched C₁₋₁₀ alkyl, cyclohexyl, phenyl-C₁₋₃ alkylene, and phenyl on the nitrogen atom.

For example, the ionic liquid comprises a halogen anion selected from the group consisting of chloride, bromide, and iodide.

Preferably, the ionic liquid is one or more selected from the group consisting of 1,3-dimethylimidazolium iodide, 1-ethyl-3-methylimidazolium iodide, 1-butyl-3-methylimidazolium iodide, 1,3-dimethylimidazolium bromide, 1-ethyl-3-methylimidazolium bromide, 1-butyl-3-methylimidazolium bromide, 1,3-dimethylimidazolium chloride, 1-ethyl-3-methylimidazolium chloride, 1-butyl-3-methylimidazolium chloride, 1-hexyl-3-methylimidazolium iodide, 1-hexyl-3-methylimidazolium bromide, 1-hexyl-3-methylimidazolium chloride, 1-methyl-3-propylimidazolium iodide, 1-methyl-3-propylimidazolium bromide, 1-methyl-3-propylimidazolium chloride, 1-decyl-3-methylimidazolium chloride, 1-decyl-3-methylimidazolium bromide, 1-decyl-3-methylimidazolium iodide, 1,3-di-*iso*-propylimidazolium chloride, 1,3-di-*iso*-propylimidazolium bromide, 1,3-di-*iso*-propylimidazolium iodide, 1,3-di-*tert-*butylimidazolium chloride, 1,3-di-*tert*-butylimidazolium bromide, 1,3-di-*tert*-butylimidazolium iodide, 1,3-dicyclohexylimidazolium chloride, 1,3-dicyclohexylimidazolium bromide, 1,3-dicyclohexylimidazolium iodide, 1-ethylpyridinium bromide, 1-ethylpyridinium chloride, and 1-ethylpyridinium iodide. For example, the ionic liquid is one or more selected from the group consisting of 1-ethyl-3-methylimidazolium bromide, 1-butyl-3-methylimidazolium bromide, and 1-ethylpyridinium bromide.

For example, the hexose diacid compound originates from biomass materials.

For example, the hexose diacid compound is one or more selected from the group consisting of glucaric acid, glucaric acid dimethyl ester, glucaric acid diethyl ester, glucaric acid dipropyl ester (*n-* and *iso*-propyl esters), glucaric acid dibutyl ester *(n-, iso-,* and *tert-butyl* esters), glucaric acid diethylene glycol ester, glucaric acid dipropylene glycol ester, glucaric acid dibutylene glycol ester, glucaric acid sodium salt, glucaric acid potassium salt, glucaric acid calcium salt, glucaro-1,4-lactone, glucaro-6,3-lactone, glucaro-1,4:6,3-dilactone, galactaric acid, galactaric acid dimethyl ester, galactaric acid diethyl ester, galactaric acid dipropyl ester(*n*- and *iso-*propyl esters), galactaric acid dibutyl ester *(n-, iso-,* and *tert-butyl* esters), galactaric acid diethylene glycol ester, galactaric acid dipropylene glycol ester, galactaric acid dibutylene glycol ester, galactaric acid sodium salt, galactaric acid potassium salt, galactaric acid calcium salt, galactaro-1,4-lactone, galactaro-6,3-lactone, galactaro-1,4:6,3-dilactone, mannaric acid, mannaric acid dimethyl ester, mannaric acid diethyl ester, mannaric acid dipropyl ester(*n*- and *iso*-propyl esters), mannaric acid dibutyl ester *(n-, iso-,* and *tert-butyl* esters), mannaric acid diethylene glycol ester, mannaric acid dipropylene glycol ester, mannaric acid dibutylene glycol ester, mannaric acid sodium salt, mannaric acid potassium salt, mannaric acid calcium salt, mannaro-1,4-lactone, mannaro-6,3-lactone, and mannaro-1,4:6,3-dilactone.

For example, the acid catalyst is one or more selected from the group consisting of hydrochloric acid, hydrobromic acid, hydroiodic acid, sulfuric acid, nitric acid, phosphoric acid, methanesulfonic acid, trifluoromethanesulfonic acid, benzenesulfonic acid, *p-*methylbenzenesulfonic acid, *p*-trifluoromethyl benzenesulfonic acid, acetic acid, trifluoroacetic acid, and phosphotungstic acid. Preferably, the acid catalyst comprises trifluoromethanesulfonic acid.

In an embodiment, no co-catalyst selected from the group consisting of lithium bromide, sodium bromide, potassium bromide, magnesium bromide, calcium bromide, barium bromide, ferric bromide, ferrous bromide, nickel bromide, copper bromide, cuprous bromide, zinc bromide, tetramethylammonium bromide, tetraethylammonium bromide, tetrapropylammonium bromide, and tetrabutylammonium bromide is added into the reaction system.

In an embodiment, no organic solvent is added into the reaction system. In another embodiment, one or more organic solvents selected from the group consisting of 1,4-dioxane, dimethyl sulfoxide, sulfolane, dimethylformamide (DMF), dimethylacetamide (DMA), diethylene glycol dimethyl ether are introduced into the reaction system.

In an embodiment, the water content in the reaction medium is below 10%, preferably below 5%.

In an embodiment, the reaction temperature of dehydration is 100-180°C.

### DETAILED DISCRIPTION

To make objects, technical details and advantages of the embodiments of the disclosure clearer, the technical solutions of the embodiments will be described below in a clearly and fully understandable way. Apparently, the described embodiments are just a part but not all of the embodiments of the disclosure. Based on the described embodiments herein, those skilled in the art can obtain other embodiment(s), without any inventive work, which should be within the scope of the disclosure.

The present disclosure can be implemented in other specific forms without departing from basic attributes of the present disclosure. It should be understood that any and all embodiments of the present disclosure can be combined with the technical features of any one or more other embodiments to obtain additional embodiments without conflict. The present disclosure comprises the additional embodiments obtained by such combination.

All the publications and patents mentioned in the present disclosure are hereby incorporated by reference in their entirety. If the use or terminology used in any publication and patent incorporated by reference conflicts with the use or terminology used in the present disclosure, the use and terminology of this disclosure shall prevail.

Unless otherwise defined, all the technical and scientific terms used in the present disclosure have the same meanings as commonly understood by one of ordinary skill in the art to which the present disclosure belongs.

The "comprise/comprising," "include/including," or "contain/containing," or similar terms are intended to specify that the elements stated before these terms encompass the elements and equivalents thereof listed after these terms, but do not preclude the other elements. The term "comprise," "include (contain)" used herein can be in open, semi-close, and close forms. In other words, the term also comprises "consisting essentially of" or "consisting of."

Unless in the working examples or otherwise indicated, all numbers expressing quantities of materials, reaction conditions, durations, quantitative properties of materials, and the like stated in the specification and claims are to be understood as modified by the term "about" in all instances. Also, it should be understood that any numerical range listed in the present application are intended to encompass all the subranges within the range and any combination of various endpoints of the range or a subrange thereof. For example, an integer of 1-20 encompasses 1, 2, 3, 4, 5, 6, 7, 8, 9, 10, 11, 12, 13, 14, 15, 16, 17, 18, 19, and 20, and also encompasses the subranges of 1-3, 1-4, 1-10, 2-4, 2-10, etc.

The description of the present disclosure should be interpreted to be consistent with the laws and principles of chemical bonding. In some cases, it is possible to remove hydrogen atom(s) to accommodate substituent(s) at given position.

In the present application, the hexose diacid compound refers to a compound having a structural formula of R₁OOC(CHOH)₄COOR₂, wherein R₁ and R₂ are each independently selected from the group consisting of hydrogen, alkali metal (*e*.*g*., lithium, sodium, or potassium), alkali earth metal (*e*.*g*., calcium or magnesium), alkyl (*e*.*g.,* C₁₋₆ alkyl), -CH₂-(CH₂)ₓ-CH₂OH (e.g., x is 0, 1, 2, 3, or 4), aralkyl (*e.g.,* benzyl and phenylethyl), and aryl *(e.g.,* phenyl). When R₁ and R₂ is hydrogen, it is a hexose diacid, *e.g.,* glucaric acid, galactaric acid, and mannaric acid.

In an embodiment, the hexose diacid compound originates from biomass materials. For example, one can extract pectin from biomass materials (*e.g.,* the peel, core and post-juicing fruit residue of fruits, such as, citrus, grapefruit, lemon, apple, pear, and hawthorn), hydrolyze the pectin by pectinase to form galacturonic acid, and then oxidize the galacturonic acid to form galacturonic acid. Alternatively, for example, one can extract alginic acid from biomass materials, *e.g.,* brown algae such as kelp and Sargassum, and then hydrolyze the alginic acid to form gulonic acid and/or mannuronic acid, which are then oxidized to form gulonic acid and/or mannuronic acid.

For example, the hexose diacid compound can be one or more selected from the group consisting of glucaric acid, glucaric acid dimethyl ester, glucaric acid diethyl ester, glucaric acid dipropyl ester (*n-* and *iso*-propyl esters), glucaric acid dibutyl ester *(n-, iso-,* and *tert-butyl* esters), glucaric acid diethylene glycol ester, glucaric acid dipropylene glycol ester, glucaric acid dibutylene glycol ester, glucaric acid sodium salt, glucaric acid potassium salt, glucaric acid calcium salt, glucaro-1,4-lactone, glucaro-6,3-lactone, glucaro-1,4:6,3-dilactone, galactaric acid, galactaric acid dimethyl ester, galactaric acid diethyl ester, galactaric acid dipropyl ester(*n*- and *iso-*propyl esters), galactaric acid dibutyl ester *(n-, iso-,* and *tert-butyl* esters), galactaric acid diethylene glycol ester, galactaric acid dipropylene glycol ester, galactaric acid dibutylene glycol ester, galactaric acid sodium salt, galactaric acid potassium salt, galactaric acid calcium salt, galactaro-1,4-lactone, galactaro-6,3-lactone, galactaro-1,4:6,3-dilactone, mannaric acid, mannaric acid dimethyl ester, mannaric acid diethyl ester, mannaric acid dipropyl ester(*n*- and *iso*-propyl esters), mannaric acid dibutyl ester *(n-, iso-,* and *tert-butyl* esters), mannaric acid diethylene glycol ester, mannaric acid dipropylene glycol ester, mannaric acid dibutylene glycol ester, mannaric acid sodium salt, mannaric acid potassium salt, mannaric acid calcium salt, mannaro-1,4-lactone, mannaro-6,3-lactone, and mannaro-1,4:6,3-dilactone.

In the present application, the 2,5-furandicarboxylic acid compound refers to a compound having a structural formula of 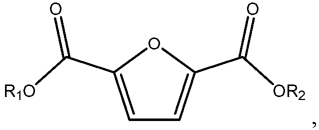 wherein R₁ and R₂ are each independently selected from the group consisting of hydrogen, alkali metal (*e.g.,* lithium, sodium, or potassium), alkali earth metal (*e.g.,* calcium or magnesium), alkyl (*e.g.,* C₁₋₆ alkyl), -CH₂-(CH₂)ₓ-CH₂OH *(e.g.,* x is 0, 1, 2, 3, or 4), aralkyl (*e.g.,* benzyl and phenylethyl), and aryl *(e.g.,* phenyl). When R₁ and R₂ are hydrogen, it is 2,5-furandicarboxylic acid.

In the present application, the "alkyl" refers to a linear or branched saturated hydrocarbon group consisting only of 1 to 20 carbon atoms. For example, C₁-C₁₀ alkyl represents an alkyl containing 1-10 carbon. Typical alkyls comprise, but are not limited to, methyl, ethyl, propyl, *iso-*propyl, *n*-butyl, *sec*-butyl, *iso*-butyl, *tert-butyl, n*-pentyl, *iso*-pentyl, *neo*-pentyl, hexyl, heptyl, octyl, nonyl, decyl, and the like. In some embodiments, it is C₁-C₆ alkyl. Representative examples of Ci-C₆ alkyl comprise, but are not limited to, methyl (C₁), ethyl (C₂), *n*-propyl (C₃), *iso*-propyl (C₃), *n-*butyl (C₄), *tert-butyl* (C₄), *sec*-butyl (C₄), *iso*-butyl (C₄), *n*-pentyl (C₅), 3-pentyl (C₅), *neo*-pentyl (C₅), 3-methyl-2-butyl (C₅), *tert*-pentyl (C₅), and *n*-hexyl (C₆), and the like.

The reaction of the present disclosure are represented by the reaction equation below, wherein the hexose diacid compound undergoes dehydration and cyclization in an ionic liquid in the presence of an acid catalyst to form a 2,5-furandicarboxylic acid (FDCA) compound.

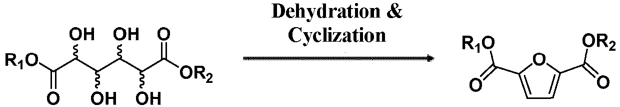

In the present application, the acid catalyst refers to a Bronsted acid capable of providing proton(s). For example, the acid catalyst is one or more selected from the group consisting of hydrochloric acid, hydrobromic acid, hydroiodic acid, sulfuric acid, nitric acid, phosphoric acid, methanesulfonic acid (MsOH), trifluoromethanesulfonic acid (TfOH), benzenesulfonic acid, *p-*methylbenzenesulfonic acid, *p*-trifluoromethyl benzenesulfonic acid, acetic acid, trifluoroacetic acid, and phosphotungstic acid.

In a preferable embodiment, the acid catalyst comprises a trifluoromethanesulfonic acid. For example, the acid catalyst is trifluoromethanesulfonic acid, or the acid catalyst is a mixture of trifluoromethanesulfonic acid with one or more selected from the group consisting of hydrochloric acid, hydrobromic acid, hydroiodic acid, sulfuric acid, nitric acid, phosphoric acid, methanesulfonic acid, benzenesulfonic acid, *p*-methylbenzenesulfonic acid, *p*-trifluoromethyl benzenesulfonic acid, acetic acid, trifluoroacetic acid, and phosphotungstic acid.

In the present application, the ionic liquid refers to a salt which is liquid at the reaction temperature and consists completely of anion and cation. The ionic liquid preferably has a melting point below 180°C, more preferably below 160°C, still preferably below 120°C, especially below 100°C. The ionic liquid comprises those which are liquid at room temperature as described in K. N. Marsh et al., Fluid Phase Equilibria 219 (2004), 93-98, and J. G. Huddleston et al., Green Chemistry 2001, 3, 156-164, for example. The yield of FDCA = (The moles of the product FDCA / The moles of the hexose diacid compound) * 100%

Unexpectedly, the inventors found that the FDCA would have relatively high yield when preparing the 2,5-furandicarboxylic acid compound by dehydration and cyclization of a hexose diacid compound using an ionic liquid comprising an anion moiety selected from halogen anions. It is believed that the ionic liquid can not only serve as reaction medium, but also provide halogen anions to help the catalysis of the reaction.

In an embodiment, the ionic liquid comprises a cation moiety selected from the group consisting of pyridinium, pyridazinium, pyrimidinium, pyrazinium, oxazinium, thiazinium, imidazolium, pyrazolium, thiazolium, isothiazolium, oxazolium, isoxazolium, or triazolium, and an anion moiety selected from halogen anions.

Persons skilled in the art will appreciate that the pyridinium, pyridazinium, pyrimidinium, pyrazinium, oxazinium, thiazinium, imidazolium, pyrazolium, thiazolium, isothiazolium, oxazolium, isoxazolium, or triazolium in the cation moiety of the ionic liquid can have substituent(s) on one or more *(e.g.,* 1, 2, or 3) nitrogen atoms. For example, the substituent is selected from the group consisting of linear or branched C₁₋₁₀ alkyl, cyclohexyl, phenylmethylene, phenylethylene, phenylpropylene, phenyl, and the like.

In an embodiment, the ionic liquid comprises a halogen anion selected from the group consisting of chloride, bromide, and iodide. For example, the ionic liquid is one or more selected from the group consisting of 1,3-dimethylimidazolium iodide, 1-ethyl-3-methylimidazolium iodide, 1-butyl-3-methylimidazolium iodide, 1,3-dimethylimidazolium bromide, 1-ethyl-3-methylimidazolium bromide, 1-butyl-3-methylimidazolium bromide, 1,3-dimethylimidazolium chloride, 1-ethyl-3-methylimidazolium chloride, 1-butyl-3-methylimidazolium chloride, 1-hexyl-3-methylimidazolium iodide, 1-hexyl-3-methylimidazolium bromide, 1-hexyl-3-methylimidazolium chloride, 1-methyl-3-propylimidazolium iodide, 1-methyl-3-propylimidazolium bromide, 1-methyl-3-propylimidazolium chloride, 1-decyl-3-methylimidazolium chloride, 1-decyl-3-methylimidazolium bromide, 1-decyl-3-methylimidazolium iodide, 1,3-di-*iso*-propylimidazolium chloride, 1,3-di-*iso*-propylimidazolium bromide, 1,3-di-*iso*-propylimidazolium iodide, 1,3-di-*tert*-butylimidazolium chloride, 1,3-di*-tert-*butylimidazolium bromide, 1,3-di-*tert*-butylimidazolium iodide, 1,3-dicyclohexylimidazolium chloride, 1,3-dicyclohexylimidazolium bromide, 1,3-dicyclohexylimidazolium iodide, 1-ethylpyridinium bromide, 1-ethylpyridinium chloride, and 1-ethylpyridinium iodide.

In a specific embodiment, the ionic liquid is one or more selected from the group consisting of 1-ethyl-3-methylimidazolium bromide, 1-butyl-3-methylimidazolium bromide, and 1-ethylpyridinium bromide.

In an embodiment, a co-catalyst is added into the reaction system. For example, the co-catalyst is one or more selected from the group consisting of lithium bromide, sodium bromide, potassium bromide, magnesium bromide, calcium bromide, barium bromide, ferric bromide, ferrous bromide, nickel bromide, copper bromide, cuprous bromide, zinc bromide, tetramethylammonium bromide, tetraethylammonium bromide, tetrapropylammonium bromide, and tetrabutylammonium bromide.

In another embodiment, no co-catalyst selected from the group consisting of lithium bromide, sodium bromide, potassium bromide, magnesium bromide, calcium bromide, barium bromide, ferric bromide, ferrous bromide, nickel bromide, copper bromide, cuprous bromide, zinc bromide, tetramethylammonium bromide, tetraethylammonium bromide, tetrapropylammonium bromide, and tetrabutylammonium bromide is added into the reaction system.

In an embodiment, no organic solvent is introduced into the reaction system.

In an embodiment, an organic solvent is introduced into the reaction system. Introducing an organic solvent may be conducive to reducing the viscosity of the ionic liquid. For example, one or more organic solvents selected from the group consisting of 1,4-dioxane, dimethyl sulfoxide, sulfolane, dimethylformamide (DMF), dimethylacetamide (DMA), and diethylene glycol dimethyl ether can be introduced into the reaction system.

Typically, the reaction temperature of the present disclosure is not higher than 180°C, *e.g.,* at 100°C, 110°C, 120°C, 130°C, 140°C, 150°C, 160°C, 170°C, or 180°C. On the one hand, too low reaction temperature is not conductive to the evaporation of water; and on the other hand, it is not conductive to shortening the reaction time. And, too high reaction temperature is likely to causing by-reaction(s). In an embodiment, the reaction temperature is 100-180°C. In a preferable embodiment, the reaction temperature 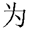 120-160°C.

It is desirable that the raw materials and reaction medium of the present disclosure have a low water content, *e.g.,* a water content below 10 wt%. In a preferable embodiment, the water content is below 5 wt%, 4 wt%, 3 wt%, 2 wt%, or 1 wt%.

It is desirable that the raw materials and reaction medium of the present disclosure have a low alcohol *(e.g.,* ethanol or ethylene glycol) content, *e.g.,* an alcohol content below 5 wt%. In a preferable embodiment, the alcohol content is below 4 wt%, 3 wt%, 2 wt%, or 1 wt%.

During or at the end of the reaction, a sample is quantitatively taken from the reaction mixture for analysis. For example, the reaction sample can be diluted for HPLC analysis by two methods. 1) The sample is neutralized with 1.0 M aqueous NaOH, further diluted with water to dissolve the ionic liquid and all the substrates and products, and then sampled for HPLC analysis. 2) The sample is directly dissolved in DMSO for analysis. The dilution for each experiment varies from 5 to 30 so that the diluted sample can have a concentration in a suitable range to be analyzed using a PDA UV detector. HPLC is used to analyze the conversion of the reaction mixture, and monitor the consumption of the substrate hexose diacid and the formation of FDCA. The method utilizes an Hi-Plex H, 300 mm x 7.7 mm HPLC column with mobile phase of 5 mmol/L aqueous dilute sulfuric acid, mobile phase rate of 0.6 ml/min, column temperature of 40°C, and injection volume of 20 µL. By such chromatography, the raw material hexose diacid has a retention time of 10.2 min, and the FDCA has a retention time of 22.5 min. The content of FDCA in each sample was accurately measured by external standard method.

The present disclosure utilizes the dehydration and cyclization of hexose diacid compound in an ionic liquid system in the presence of an acid catalyst to prepare the 2,5-furandicarboxylic acid (FDCA), wherein using the ionic liquid as reaction medium replaces or reduces the use of other polar solvents. In the reaction, high conversion and FDCAyield (with maximum FDCAyield of up to 93.5%) can be achieved without addition of halides. The reaction can be carried out at ambient pressure, and no pressure reduction is required during the reaction process. The reaction is highly effective, and can be completed at 120-160°C for 1-5 h. The reaction can be carried out with mild reaction conditions, high selectivity of FDCA, high product purity. The whole production process is less pollutive to the environment, and suitable for commercial production.

### EXAMPLES

The following examples are provided to further describe the present disclosure. It should also be understood that these examples are only for the purpose of illustrating the present disclosure, rather than limiting the scope thereof. The experimental methods without specific conditions in the following examples can be carried out according to the conventional conditions of this type of reaction or the conditions suggested by the manufacturer, and the experimental materials and reagents used can be commercially available unless otherwise specified.

In Experiments 1-36 below, glucaric acid monopotassium salt was used as the hexose diacid compound material to undergo dehydration and cyclization in accordance with the reaction conditions listed in Tables 1-4, using 1-ethyl-3-methylimidazolium bromide, 1-butyl-3-methylimidazolium bromide, or 1-ethyl pyridinium bromide as ionic liquid, and trifluoromethanesulfonic acid, methanesulfonic acid, or sulfuric acid as acid catalyst, to prepare the 2,5-furandicarboxylic acid (FDCA).

Taking Experiment 1 as example, glucaric acid monopotassium salt (0.099 g, 0.4 mmol), 1-ethyl-3-methylimidazolium bromide (0.61 g, 3.2 mmol), and trifluoromethanesulfonic acid (0.36 g, 2.4 mmol) were added into a thick-wall pressure-proof tube. The reaction mixture was heated to 120°C for 1h, and 1M *aq* NaOH was added into the reaction system at the end of the reaction. The reaction system was adjusted to pH 8-10. After the reaction solution was completely dissolved, a small amount of solution was taken as sample, diluted with 5 mmol *aq* H₂SO₄, and subjected to HPLC analysis for the conversion of the reaction. The FDCA yield was 86.7%.

**Table 1: Synthesis Of FDCA In 1-Ethyl-3-Methylimidazolium Bromide In The Presence Of Trifluoromethanesulfonic Acid Catalyst**

| Experimental result of synthesis of FDCA in ionic liquid | | | | | | | |
|---|---|---|---|---|---|---|---|
| Exper iment | Substrate/D-Glucaric Acid Monopotassiu m Salt | TfOH Equival ent(S) | 1-Ethyl-3-Methylimidazoli um Bromide Equivalent | Mass Ratio Of Ionic Liquid/Substra te | Reaction Temperatur e (°C) | Reactio n Time (H) | FDCA Yield (%) |
| 1 | 0.099 g | 6.0 | 8.0 | 6.1 | 120°C | 1 | 86.7 |
| 2 | 0.099 g | 6.0 | 8.0 | 6.1 | 120°C | 2 | 89.3 |
| 3 | 0.099 g | 6.0 | 8.0 | 6.1 | 120°C | 3 | 86.9 |
| 6 | 0.495 g | 6.0 | 8.0 | 6.1 | 120°C | 5 | 93.5 |
| 7 | 0.099 g | 6.0 | 8.0 | 6.1 | 150°C | 0.5 | 85.9 |
| 8 | 0.099 g | 6.0 | 8.0 | 6.1 | 150°C | 1 | 87.7 |
| 9 | 0.099 g | 6.0 | 8.0 | 6.1 | 150°C | 5 | 87.0 |
| 10 | 0.099 g | 3.0 | 8.0 | 6.1 | 120°C | 3 | 47.8 |
| 11 | 0.099 g | 4.0 | 8.0 | 6.1 | 120°C | 3 | 70.6 |
| 12 | 0.099 g | 5.0 | 8.0 | 6.1 | 120°C | 3 | 87.6 |
| 13 | 0.099 g | 7.0 | 8.0 | 6.1 | 120°C | 3 | 77.0 |
| 14 | 0.099 g | 8.0 | 8.0 | 6.1 | 120°C | 3 | 77.9 |
| 15 | 0.099 g | 5.0 | 5.0 | 3.8 | 120°C | 5 | 81.5 |
| 16 | 0.099 g | 5.0 | 6.0 | 4.6 | 120°C | 5 | 83.4 |
| 17 | 0.099 g | 5.0 | 7.0 | 5.3 | 120°C | 5 | 85.7 |
| 18 | 0.099 g | 6.0 | 8.0 | 6.1 | 120°C | 5 | 81.5^{[a]} |
| 19 | 0.099 g | 6.0 | 8.0 | 6.1 | 120°C | 5 | 67.6^{[b]} |
| 20 | 0.099 g | 6.0 | 8.0 | 6.1 | 120°C | 5 | 33.4^{[c]} |
| 21 | 0.099 g | 6.0 | 8.0 | 6.1 | 120°C | 5 | 14.9^{[d]} |
| 22 | 0.099 g | 6.0 | 8.0 | 6.1 | 120°C | 5 | 80.6^{[e]} |
| 23 | 0.099 g | 6.0 | 8.0 | 6.1 | 120°C | 5 | 78.6^{[f]} |
| 24 | 0.099 g | 6.0 | 8.0 | 6.1 | 120°C | 3 | 80.0^{[g]} |

| | | | | | | | |
|---|---|---|---|---|---|---|---|
| Note: [a], [b], [c] Adding 5 wt%, 10 wt%, 20 wt% of water into the reaction system, respectively; [d] Adding 4.48 equivalent of ethylene glycol into the reaction system; [e], [f], [g] Adding 0.3 mL, 0.5 mL, 1.0 mL of sulfolane into the reaction system, respectively. | | | | | | | |

**Table 2: Synthesis Of FDCA In 1-Ethyl-3-Methylimidazolium Bromide Using Methanesulfonic Acid/Sulfuric Acid**

| Experimental result of synthesis of FDCA in ionic liquid | | | | | | | |
|---|---|---|---|---|---|---|---|
| Exper iment | Substrate/D-Glucaric Acid Monopotassiu m Salt | Acid Catalyst Equivalent | 1-Ethyl-3-Methylimidazoli um Bromide Equivalent | Mass Ratio Of Ionic Liquid/Substr ate | Reaction Temperat ure (°C) | Reactio n Time (H) | FDCA Yield (%) |
| 4 | 0.099 g | Methane sulfonic acid 10.0 | 8.0 | 6.1 | 120°C | 5 | 31.6 |
| 5 | 0.099 g | Sulfuric acid 8.0 | 8.0 | 6.1 | 120°C | 5 | 8.2 |

**Table 3: Synthesis Of FDCA In 1-Ethylpyridinium Bromide Using Trifluoromethanesulfonic Acid**

| Experimental Result Of Synthesis Of FDCA In Ionic Liquid | | | | | | | |
|---|---|---|---|---|---|---|---|
| Exper iment | Substrate/D-Glucaric Acid Monopotassiu m Salt | TfOH Equivale nt | 1-Ethylpyridinium Bromide Equivalent | Mass Ratio Of Ionic Liquid/Substra te | Reaction Temperatur e (°C) | Reaction Time (H) | FDCA Yield (%) |
| 25 | 0.099 g | 6.0 | 4.0 | 3.0 | 120°C | 5 | 78.7 |
| 26 | 0.099 g | 6.0 | 5.0 | 3.8 | 120°C | 5 | 83.5 |
| 27 | 0.099 g | 6.0 | 6.0 | 4.5 | 120°C | 5 | 87.1 |
| 28 | 0.099 g | 6.0 | 7.0 | 5.3 | 120°C | 5 | 87.4 |
| 29 | 0.099 g | 4.0 | 5.0 | 3.8 | 120°C | 5 | 89.7 |
| 30 | 0.099 g | 5.0 | 5.0 | 3.8 | 120°C | 5 | 86.0 |

**Table 4: Synthesis Of FDCA In 1-Butyl-3-Methylimidazolium Bromide Using Trifluoromethanesulfonic Acid**

| Experimental Result Of Synthesis Of FDCA In Ionic Liquid | | | | | | | |
|---|---|---|---|---|---|---|---|
| Exper iment | Substrate/D-Glucaric Acid Monopotassiu m Salt | TfOH Equival ent | 1-Butyl-3-Methylimidazoli um Bromide Equivalent | Mass Ratio Of Ionic Liquid/Substra te | Reaction Temperatur e (°C) | Reaction Time (H) | FDCA Yield (%) |
| 31 | 0.099 g | 6.0 | 4.0 | 3.5 | 120°C | 5 | 75.5 |
| 32 | 0.099 g | 6.0 | 5.0 | 4.4 | 120°C | 5 | 81.7 |
| 33 | 0.099 g | 6.0 | 6.0 | 5.3 | 120°C | 5 | 83.8 |
| 34 | 0.099 g | 6.0 | 8.0 | 7.0 | 120°C | 5 | 90.1 |
| 35 | 0.099 g | 4.0 | 5.0 | 4.4 | 120°C | 5 | 85.6 |
| 36 | 0.099 g | 5.0 | 5.0 | 4.4 | 120°C | 5 | 85.4 |

A plurality of experiments were carried out in the present disclosure, wherein, when the mass ratio of the ionic liquid to the substrate glucaric acid potassium salt was 6.1, TfOH was 6.0 eq., and the reaction was carried out at 120°C for 5 h, the maximum FDCA yield could be up to 93.5% (Experiment-6). Under the same experimental conditions, when the reaction was heated to a temperature of 150°C for 0.5 h, the FDCA yield could achieve 85.9% (Experiment-7). When the mass ratio of the ionic liquid to the substrate glucaric acid potassium salt was adjusted to 3.8, TfOH was 5.0 eq., and the reaction was carried out at 120°C for 5 h, the FDCA yield could be up to 81.5% (Experiment-15). When 5 wt% H₂O was added into the reaction system, and the reaction was carried at 120°C for 5 h, the FDCA yield could still be up to 81.5% (Experiment-18). Adding the polar solvent sulfolane into the reaction system did not influence the reaction effect (Experiments 22-24). Using other ionic liquids in the reaction, it could still achieve a relatively high FDCA conversion (Experiments 25-36).

As shown in Table 5 below, in Experiment 37-38, glucaric acid monopotassium salt was used as the hexose diacid compound material to undergo dehydration and cyclization in accordance with the reaction conditions listed in Table 5, using 1-ethyl-3-methylimidazolium bisulfate as ionic liquid, and sulfuric acid as acid catalyst, to prepare 2,5-furandicarboxylic acid (FDCA).

**Table 5: Synthesis Of FDCA In 1-Ethyl-3-Methylimidazolium Bisulfate Using Sulfuric Acid**

| Experi ment | Substrate/D-Glucaric Acid Monopotassiu m Salt | H₂SO₄ Equivale nt | 1-Ethyl-3-Methylimidazolium Bisulfate Equivalent | Mass Ratio Of Ionic Liquid/Substr ate | Reactio n Temper ature | Reacti on Time | Yield % (FDCA) |
|---|---|---|---|---|---|---|---|
| 37 | 0.099 g | 2.0 eq | 6.0 eq | 5.0 | 120°C | 5h | 9.3 |
| 38 | 0.099 g | 3.0 eq | 6.0 eq | 5.0 | 120°C | 5h | 17.4 |

Examples 37-38 utilized 1-ethyl-3-methylimidazolium bisulfate and sulfuric acid, which have significantly reduced FDCA yield.

As shown in Table 6 below, in Experiment 39-40, glucaric acid monopotassium salt was used as the hexose diacid compound material to undergo dehydration and cyclization in accordance with the reaction conditions listed in Table 6, using 1-ethyl-3-methylimidazolium hexafluorophosphate as ionic liquid, and trifluoromethanesulfonic acid as acid catalyst, to prepare 2,5-furandicarboxylic acid (FDCA).

**Table 6: Synthesis Of FDCA In 1-Ethyl-3-Methylimidazolium Hexafluorophosphate Using Trifluoromethanesulfonic Acid**

| Exper iment | Substrate/D-Glucaric Acid Monopotassi um Salt | Tfoh Equival ent | 1-Ethyl-3-Methylimidazolium Hexafluorophosphat e | Mass Ratio Of Ionic Liquid/Substr ate | Reactio n Temper ature | Reacti on Time | Yield % (FDCA) |
|---|---|---|---|---|---|---|---|
| 39 | 0.099 g | 4.0 eq | 6.0 eq | 6.2 | 120°C | 5h | 0.4 |
| 40 | 0.099 g | 5.0 eq | 6.0 eq | 6.2 | 120°C | 5h | 0.3 |

As shown in Table 7 below, in Experiment 41-42, glucaric acid monopotassium salt was used as the hexose diacid compound material to undergo dehydration and cyclization in accordance with the reaction conditions listed in Table 7, using 1-ethyl-3-methylimidazolium tetrafluoroborate as ionic liquid, and trifluoromethanesulfonic acid as acid catalyst, to prepare 2,5-furandicarboxylic acid (FDCA).

**Table 7: Synthesis Of FDCA In 1-Ethyl-3-Methylimidazolium Tetrafluoroborate Using Trifluoromethanesulfonic Acid**

| Exper iment | Substrate/D-Glucaric Acid Potassium Salt | Tfoh Equivalen t | 1-Ethyl-3-Methylimidazolium Tetrafluoroborate Equivalent | Mass Ratio Of Ionic Liquid/Subst rate | Reactio n Temper ature | Reacti on Time | Yield % (FDCA) |
|---|---|---|---|---|---|---|---|
| 41 | 0.099 g | 4.0 eq | 6.0 eq | 4.7 | 120°C | 5h | 1.7 |
| 42 | 0.099 g | 5.0 eq | 6.0 eq | 4.7 | 120°C | 5h | 3.6 |

As shown in Table 8 below, in Experiment 43-44, glucaric acid monopotassium salt was used as the hexose diacid compound material to undergo dehydration and cyclization, using 1-ethyl-3-methylimidazolium trifluoromethane sulfonate as ionic liquid, and trifluoromethanesulfonic acid as acid catalyst, to prepare 2,5-furandicarboxylic acid (FDCA).

**Table 8: Synthesis Of FDCA In 1-Ethyl-3-Methylimidazolium Trifluoromethane Sulfonate Using Trifluoromethanesulfonic Acid**

| Exper iment | Substrate/D-Glucaric Acid Potassium Salt | Tfoh Equivale nt | 1-Ethyl-3-Methylimidazolium Trifluoro Methane Sulfonate Equivalent | Mass Ratio Of Ionic Liquid/Substr ate | Reactio n Temper ature | Reacti on Time | Yield % (FDCA) |
|---|---|---|---|---|---|---|---|
| 43 | 0.099 g | 4.0 eq | 6.0 eq | 6.3 | 120°C | 5 h | 24.0 |
| 44 | 0.099 g | 5.0 eq | 6.0 eq | 6.3 | 120°C | 5 h | 37.6 |

Experiments 39-44 utilized trifluoromethanesulfonic acid as catalyst, and 1-ethyl-3-methylimidazolium hexafluorophosphate, 1-ethyl-3-methylimidazolium tetrafluoroborate, or 1-ethyl-3-methylimidazolium trifluoro methane sulfonate as ionic liquid, and the yield of FDCA prepared in these experiments were significantly lower than that prepared by using 1-ethyl-3-methylimidazolium bromide in Table 1.

Compared with the existing technology, the present disclosure has one or more advantages of:
1) The reaction can be carried at ambient pressure, can produce FDCA with high yield, and does not need to be carried out under vacuum. At the same time, the reaction will not produce a large amount of hydrogen halide vapor at high temperature, which will lead to the need to use reactors such as special pressure-resistant devices, and has low equipment requirements;
2) The ionic liquid is used as both catalyst and solvent in the reaction, which can avoid the need of adding some strong polar aprotic solvents (such as sulfolane, dimethyl sulfoxide, and 1,4- dioxane) in the reaction in the prior art, and the whole production process is less harmful to the environment;
3) The reaction takes a short time, and commonly can be completed in 1-5 h;
4) It has simple operation, good FDCA selectivity, high yield with maximum yield of up to 85-95%, and good reaction stability, which is very suitable for commercial production;
5) The product FDCA obtained by post-treatment has high purity; and
6) During the reaction process, the reaction progress can be controlled by addition of acid dropwise, that is, the reaction is a feed-controlled reaction in nature, and has low safety risk in the production.

As shown in Table 9 below, in Experiments 45-47, D-glucaric acid monopotassium salt was used as raw material to undergo dehydration and cyclization, using sulfolane as solvent, NaBr or LiBr as additive, in the presence of TfOH acid catalyst, to give 2,5-furandicarboxylic acid, wherein the reaction temperature was 120-160°C.

**Table 9: Synthesis Of FDCA Using Trifluoromethanesulfonic Acid In The Presence Of Sodium Bromide/Potassium Bromide**

| Experime nt | Substrate/D-Glucaric Acid Monopotassi um Salt | Tfoh Equivale nt | Bromide Equivale nt | Bromide/Substr ate Mass Ratio | Sulfola ne Volume | Reaction Temperatu re | Reacti on Time | Yield % (FDC A) |
|---|---|---|---|---|---|---|---|---|
| 45 | 0.099 g | 10.0 eq | 8.0 eq^{[h]} | 3.3 | 1.0 mL | 120°C | 2 h | 86.6 |
| 46 | 0.099 g | 10.0 eq | 8.0 eq^{[h]} | 3.3 | 1.0 mL | 150°C | 1 h | 83.9 |
| 47 | 0.099 g | 10.0 eq | 16.0 eq^{[i]} | 5.6 | 1.0 mL | 160°C | 1 h | 91.0 |

| | | | | | | | | |
|---|---|---|---|---|---|---|---|---|
| NOTE: [H] Sodium bromide; [i] Lithium bromide. | | | | | | | | |

The advantages and disadvantages of the technical solutions of Experiments are as follows: 1) The reaction is necessarily carried out in a pressure- and corrosion-resistance equipment due to the generation of HX at high temperature, that is, having high requirement for equipment; 2) Bronsted acid and halide are used in a large amount in the reaction; and 3) a large amount of acidic waste gas, waste material, etc. will be produced.

In Experiments 48-51, D-glucaric acid monopotassium salt was used as raw material to undergo dehydration and cyclization, using sulfolane as solvent, tetrabutylammonium bromide (TBAB), tetramethylammonium bromide (TMAB), or tetraethylammonium bromide (TEAB) as additive, in the presence of TfOH acid catalyst, to give 2,5-furandicarboxylic acid, wherein the reaction temperature was 120°C.

**Table 10: Synthesis Of FDCA Using Trifluoromethanesulfonic Acid In The Presence Of Tetrabutylammonium Bromide, Tetramethylammonium Bromide, Or Tetraethylammonium Bromide**

| Experim ent | Substrate/D-Glucaric Acid Monopotass ium Salt | Tfoh Equival ent | Bromid e Equival ent | Mass Ratio Of Bromide/Subs trate | Solvent/Sulfo lane | Reaction Temperat ure | Reacti on Time | Yield % (FDC A) |
|---|---|---|---|---|---|---|---|---|
| 48 | 0.099 g | 6.0 eq | 10.0 eq^{[j]} | 13.0 | 1.0 mL | 120°C | 5 h | 81.5 |
| 49 | 0.099 g | 6.0 eq | 8.0 eq^{[k]} | 5.0 | 1.0 mL | 120°C | 5 h | 86.0 |
| 50 | 0.099 g | 10.0 eq | 8.0 eq^{[k]} | 5.0 | 1.0 mL | 120°C | 5 h | 81.3 |
| 51 | 0.099 g | 8.0 eq | 8.0 eq^{[l]} | 6.8 | 0.5 mL | 120°C | 5 h | 90.6 |

| | | | | | | | | |
|---|---|---|---|---|---|---|---|---|
| Note: [j] Tetrabutylammonium bromide; [k] Tetramethylammonium bromide; [l] Tetraethylammonium bromide. | | | | | | | | |

The advantages and disadvantages of the technical solutions of Experiments are as follows: 1) Bronsted acid and halide are used in a large amount in the reaction; and 2) a large amount of acidic waste gas, waste material, etc. will be produced.

In Experiments 52-54, galactaric acid was used as raw material to undergo dehydration and cyclization using 1-ethyl-3-methylimidazolium bromide as ionic liquid in the presence of TfOH acid catalyst to give 2,5-furandicarboxylic acid, wherein the reaction temperature was 120°C.

**Table 11: Synthesis Of FDCA With Galactaric Acid As Raw Material Using Trifluoromethanesulfonic Acid In 1-Ethyl-3-Methylimidazolium Bromide**

| Exper iment | Substrate/Gal actaric Acid | Tfoh Equivalen t | 1-Ethyl-3-Methylimidazoliu m Bromide Equivalent | Mass Ratio Of Ionic Liquid/Subst rate | Reaction Temperat ure | Reaction Time | Yield % (FDCA) |
|---|---|---|---|---|---|---|---|
| 52 | 0.084 g | 2.0 eq | 8.0 eq | 6.2 | 120°C | 5 h | 40.7 |
| 53 | 0.084 g | 4.0 eq | 8.0 eq | 6.2 | 120°C | 5 h | 60.1 |
| 54 | 0.084 g | 5.0 eq | 8.0 eq | 6.2 | 120°C | 5 h | 61.2 |

Experiments 52-54 indicate that galactaric acid can replace D-glucaric acid for use in the method of the present disclosure to synthesize FDCA.

Although the present disclosure has been described hereinbefore using specific embodiments and examples, many changes and modifications will be obvious to those skilled in the art. Thus, the described embodiments are considered in all respects to illustrative, rather than limitative. Thus, the scope of the present disclosure is defined by the appended claims, other than the foregoing description. All modifications within equivalent meanings and ranges of the claims are to be encompassed in the protection scope of the present disclosure.

## Claims

1. A method for preparing a 2,5-furandicarboxylic acid (FDCA) compound, comprising:
providing a hexose diacid compound, an acid catalyst, and ionic liquid; and
dehydrating the hexose diacid compound in the ionic liquid in the presence of the acid catalyst to give the 2,5-furandicarboxylic acid compound;
wherein the ionic liquid comprises a cation moiety selected from the group consisting of pyridinium, pyridazinium, pyrimidinium, pyrazinium, oxazinium, thiazinium, imidazolium, pyrazolium, thiazolium, isothiazolium, oxazolium, isoxazolium, or triazolium, and an anion moiety selected from halogen anions.

2. The method of claim 1, wherein the ionic liquid has a substituent selected from the group consisting of linear or branched C₁₋₁₀ alkyl, cyclohexyl, phenyl-C₁₋₃ alkylene, and phenyl on a nitrogen atom.

3. The method of claim 1 or 2, wherein the ionic liquid comprises a halogen anion selected from the group consisting of chloride, bromide, and iodide.

4. The method of claim 3, wherein the ionic liquid is one or more selected from the group consisting of 1,3-dimethylimidazolium iodide, 1-ethyl-3-methylimidazolium iodide, 1-butyl-3-methylimidazolium iodide, 1,3-dimethylimidazolium bromide, 1-ethyl-3-methylimidazolium bromide, 1-butyl-3-methylimidazolium bromide, 1,3-dimethylimidazolium chloride, 1-ethyl-3-methylimidazolium chloride, 1-butyl-3-methylimidazolium chloride, 1-hexyl-3-methylimidazolium iodide, 1-hexyl-3-methylimidazolium bromide, 1-hexyl-3-methylimidazolium chloride, 1-methyl-3-propylimidazolium iodide, 1-methyl-3-propylimidazolium bromide, 1-methyl-3-propylimidazolium chloride, 1-decyl-3-methylimidazolium chloride, 1-decyl-3-methylimidazolium bromide, 1-decyl-3-methylimidazolium iodide, 1,3-di-*iso*-propylimidazolium chloride, 1,3-di-*iso*-propylimidazolium bromide, 1,3-di-*iso*-propylimidazolium iodide, 1,3-di-*tert-*butylimidazolium chloride, 1,3-di-*tert*-butylimidazolium bromide, 1,3-di-*tert*-butylimidazolium iodide, 1,3-dicyclohexylimidazolium chloride, 1,3-dicyclohexylimidazolium bromide, 1,3-dicyclohexylimidazolium iodide, 1-ethylpyridinium bromide, 1-ethylpyridinium chloride, and 1-ethylpyridinium iodide.

5. The method of claim 4, wherein the ionic liquid is one or more selected from the group consisting of 1-ethyl-3-methylimidazolium bromide, 1-butyl-3-methylimidazolium bromide, and 1-ethylpyridinium bromide.

6. The method of any one of claims 1-5, wherein the hexose diacid compound originates from biomass materials.

7. The method of any one of claims 1-5, wherein the hexose diacid compound is one or more selected from the group consisting of glucaric acid, glucaric acid dimethyl ester, glucaric acid diethyl ester, glucaric acid dipropyl ester (*n-* and *iso*-propyl esters), glucaric acid dibutyl ester *(n-, iso-,* and *tert-butyl* esters), glucaric acid diethylene glycol ester, glucaric acid dipropylene glycol ester, glucaric acid dibutylene glycol ester, glucaric acid sodium salt, glucaric acid potassium salt, glucaric acid calcium salt, glucaro-1,4-lactone, glucaro-6,3-lactone, glucaro-1,4:6,3-dilactone, galactaric acid, galactaric acid dimethyl ester, galactaric acid diethyl ester, galactaric acid dipropyl ester(*n*- and *iso*-propyl esters), galactaric acid dibutyl ester *(n-, iso-,* and *tert-butyl* esters), galactaric acid diethylene glycol ester, galactaric acid dipropylene glycol ester, galactaric acid dibutylene glycol ester, galactaric acid sodium salt, galactaric acid potassium salt, galactaric acid calcium salt, galactaro-1,4-lactone, galactaro-6,3-lactone, galactaro-1,4:6,3-dilactone, mannaric acid, mannaric acid dimethyl ester, mannaric acid diethyl ester, mannaric acid dipropyl ester(*n*- and *iso*-propyl esters), mannaric acid dibutyl ester *(n-, iso-,* and *tert-butyl* esters), mannaric acid diethylene glycol ester, mannaric acid dipropylene glycol ester, mannaric acid dibutylene glycol ester, mannaric acid sodium salt, mannaric acid potassium salt, mannaric acid calcium salt, mannaro-1,4-lactone, mannaro-6,3-lactone, and mannaro-1,4:6,3-dilactone.

8. The method of any one of claims 1-7, wherein the acid catalyst is one or more selected from the group consisting of hydrochloric acid, hydrobromic acid, hydroiodic acid, sulfuric acid, nitric acid, phosphoric acid, methanesulfonic acid, trifluoromethanesulfonic acid, benzenesulfonic acid, *p*-methylbenzenesulfonic acid, *p*-trifluoromethyl benzenesulfonic acid, acetic acid, trifluoroacetic acid, and phosphotungstic acid.

9. The method of claim 8, wherein the acid catalyst comprises trifluoromethanesulfonic acid.

10. The method of any one of claims 1-9, wherein no co-catalyst selected from the group consisting of lithium bromide, sodium bromide, potassium bromide, magnesium bromide, calcium bromide, barium bromide, ferric bromide, ferrous bromide, nickel bromide, copper bromide, cuprous bromide, zinc bromide, tetramethylammonium bromide, tetraethylammonium bromide, tetrapropylammonium bromide, and tetrabutylammonium bromide is added into the reaction system.

11. The method of any one of claims 1-10, wherein no organic solvent is added into the reaction system.

12. The method of any one of claims 1-10, wherein one or more organic solvents selected from the group consisting of 1,4-dioxane, dimethyl sulfoxide, sulfolane, dimethylformamide (DMF), dimethylacetamide (DMA), and diethylene glycol dimethyl ether are introduced into the reaction system.

13. The method of any one of claims 1-12, wherein a reaction medium has a water content below 10%.

14. The method of claim 13, wherein the reaction medium has a water content below 5%.

15. The method of any one of claims 1-14, wherein a reaction temperature of dehydration is 100-180°C.
